# EUROPEAN PATENT APPLICATION

(11) **EP 2 338 444 A1**
(43) Date of publication of application: **29.06.2011**
(21) Application number: 10014956.6
(22) Date of filing: 24.11.2010
(51) Int. Cl.: A61F 2/38

(54) **Method for manufacturing an orthopedic implant**

(30) Priority: 23.12.2009 US 645946
(71) Applicant: Zimmer GmbH, 8404 Winterthur (CH)
(72) Inventor: Donno, Cosimo, 8422 Pfungen (CH)
(74) Representative: Manitz, Finsterwald & Partner GbR

(57) **Abstract**

The present disclosure relates to a method for manufacturing an orthopedic implant. The method includes providing a blank (18) adapted to be formed into an orthopedic implant, forging the blank to form an intermediate component (24), and bending the forged component to form a pre-finished component (38).

## Description

### BACKGROUND

### 1. Field of the Disclosure

The present disclosure relates to a method for manufacturing an orthopedic implant and, particularly, to a method for manufacturing a femoral component of a knee joint prosthesis.

### 2. Description of the Related Art

Knee arthroplasty is used to partially or totally replace knee joints which have been damaged by trauma or disease such as arthritis. In total knee arthroplasty, the damaged surfaces of the knee joint are surgically removed and replaced with artificial surfaces. Particularly, the distal articulating surface of the femur is replaced by a prosthetic femoral component, and the proximal articulating surface of the tibia is replaced by a prosthetic tibial component, thereby providing a prosthetic knee joint. In bone-conserving surgical procedures where the femur is simply re-shaped rather than removed, resurfacing femoral components are used. Femoral components of knee joint prostheses are typically formed of a rigid metal and cast into the desired shapes which may have complex geometries.

### SUMMARY

One object of this disclosure is to provide a simple and economic method for manufacturing an orthopedic implant.

Accordingly, the present disclosure provides a method that comprises: providing a blank adapted to be formed into the orthopedic implant; carrying out a forging operation on the blank so as to form an intermediate component; and carrying out a bending operation on the intermediate component so as to form a pre-finished component.

To manufacture an orthopedic implant, the method of the present disclosure carries out the forging operation before the bending operation. Forging before bending has the advantage that topological features of the orthopedic implant such as cement pockets at an anchoring side of the orthopedic implant can be applied to the unbent blank and thus with easy access. In addition, a forged orthopedic implant has a high fatigue strength which is particularly beneficial to resurfacing implants.

In one aspect, the intermediate component is of substantially planar shape.

In another aspect, the pre-finished component has a shape substantially corresponding to the shape of the orthopedic implant.

In yet another aspect, the forging operation includes forming the underlying geometrical structure of the orthopedic implant, particularly including at least one of lateral and medial condyle portions of a femoral component of a knee joint prosthesis.

In still another aspect, the forging operation includes forming at least one depression in a first side of the blank. The depression is adapted to serve as a cement pocket in the orthopedic implant. The first side is adapted to serve as an anchoring side of the orthopedic implant adapted to be arranged at a resection surface of a bone.

The shape and size of the depression in the intermediate component may be different from the shape and size of the cement pocket in the orthopedic implant. Therefore, the formation of the depression may be such that the effect of the bending operation on the shape and size of the depression is considered or accounted for.

In yet another aspect, the bending operation includes forming a substantially rounded anchoring side of the orthopedic implant and/or the orthopedic implant has a substantially uniform thickness.

In another exemplary aspect, the blank is a pre-forged blank.

In a specific aspect, the forging operation is a die forging operation.

In another specific aspect, the bending operation is a die bending operation.

In yet another specific aspect, the method comprises at least one of: deburring, polishing, and cleaning the pre-finished component.

In still another aspect, the blank and thus the orthopedic implant are formed from a material selected from: titanium and titanium alloy.

In accordance with another embodiment of the invention, an orthopedic component made in accordance with the above-described method is provided.

The present disclosure further relates to a method for implanting an orthopedic implant comprising preparing a bone to receive the orthopedic implant, and particularly to a method for implanting a femoral component of a knee joint prosthesis comprising preparing a femur to receive the femoral component. In one aspect, the bone is prepared such that it is substantially rounded when receiving the orthopaedic implant.

### BRIEF DESCRIPTION OF THE DRAWINGS

The above-mentioned and other features and advantages of this disclosure, and the manner of attaining them, will become more apparent and the disclosure itself will be better understood by reference to the following description of an embodiment of the disclosure taken in conjunction with the accompanying drawings, wherein:

Fig. 1 is a perspective view of a femur;

Fig. 2 is a schematic representation of a forging operation on a blank;

Fig. 3 is a perspective view of an intermediate component;

Fig. 4 is a schematic representation of a bending operation on the intermediate component of Fig. 3;

Fig. 5 is a perspective view of a pre-finished component; and

Figs. 6a, b are cross-sectional views of femoral components of a knee joint prosthesis according the present disclosure and to the prior art, respectively.

The following description is merely exemplary in nature and is not intended to limit the present disclosure, application, or uses. It should be understood that throughout the drawings, identical reference numerals indicate the same or corresponding parts and features.

### DETAILED DESCRIPTION

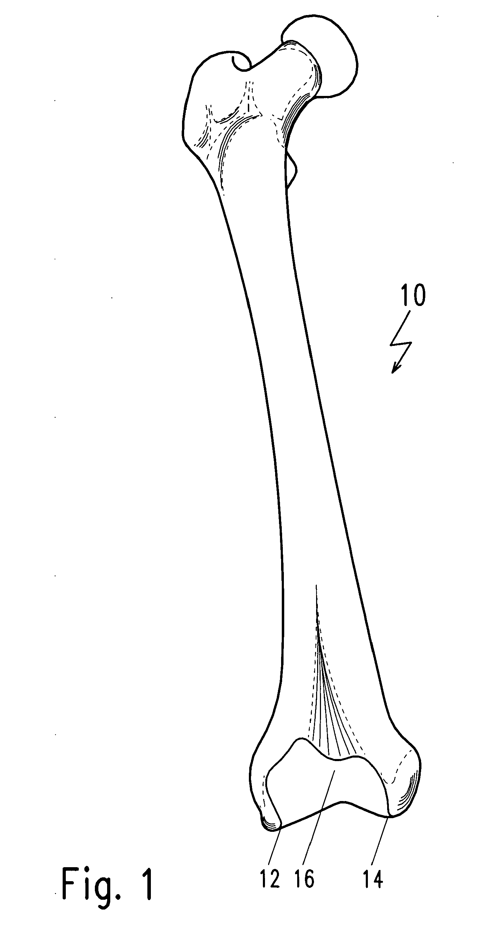

Right femur 10 shown in Fig. 1 includes lateral and medial condyles 12 and 14 which are in articulating contact to a tibia head (not shown) and facies patellaris 16 which is in articulating contact with a patella (not shown). A resurfacing femoral component of a knee joint prosthesis is adapted for attachment to a resected femur to replace lateral condyle 12, medial condyle 14 and facies patellaris 16. In partial knee arthroplasty, also called unicompartmental knee arthroplasty, only one of lateral condyle 12 and medial condyle 14 is replaced.

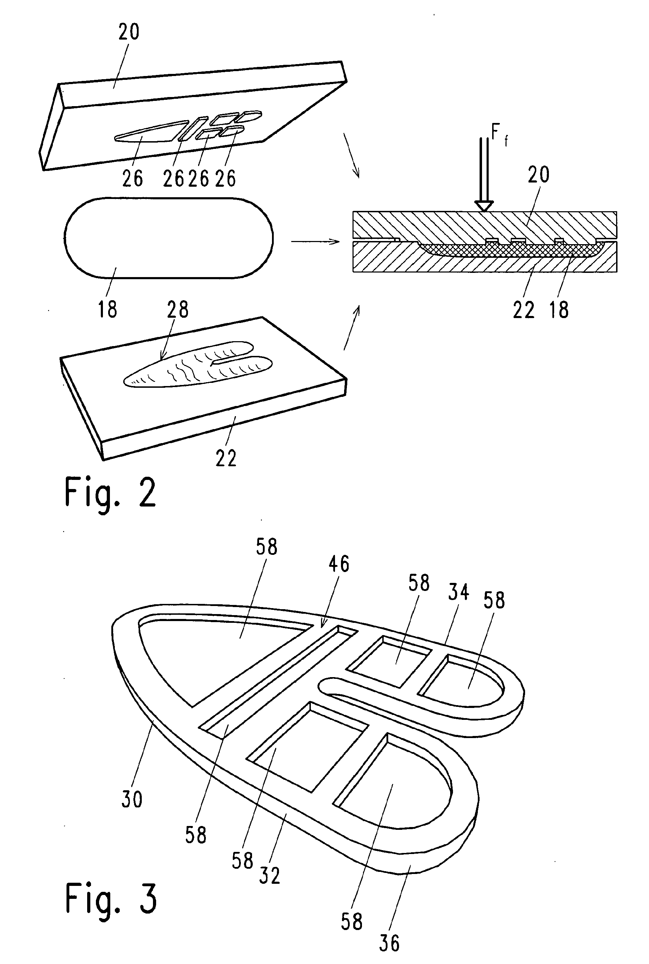

Figs. 2-5 show the manufacturing steps of a resurfacing femoral component.

In a first step, as shown in Fig. 2, blank 18, made of e.g. titanium, is provided. The blank 18 is pre-forged, which is to say it has a size and shape adapted to be formed into the final femoral component.

In a second step, blank 18 is forged. Blank 18 is positioned between male and female die plates 20, 22 which are pressed against each other by a force F_{f} so as to form, either by cutting or a non-cutting deformation, a substantially planar intermediate component 24 (Fig. 3). The male die plate 20 includes protrusions 26 so as to form depressions 58 in upper side 46 of intermediate component 24 which are adapted to serve as cement pockets in an anchoring side of the final femoral component. In general, male die plate 20 is adapted to form the topographic features of the final femoral component. Female die plate 22 comprises cavity 28 having a shape which pre-defines the overall (unbent or flattened) shape of the final femoral component and, particularly, of anterior portion 30, distal portion 32 and lateral and medial condyle portions 34, 36 respectively.

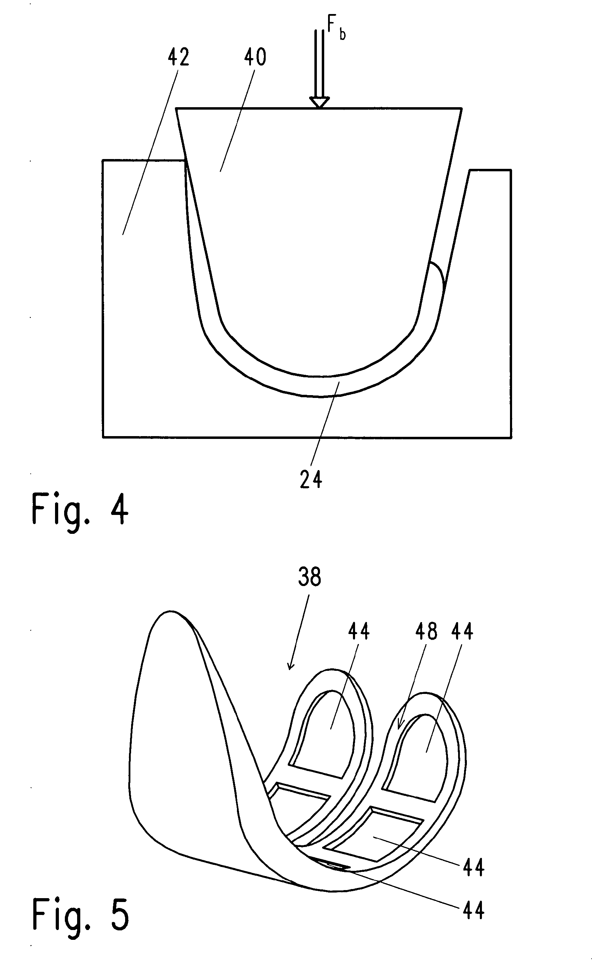

In a third step, as shown in Fig. 4, intermediate component 24 is bent by a force F_{b} so as to form a pre-finished component 38 (Fig. 5). The bending is carried out by a bending die including punch 40 and die plate 42. Pre-finished component 38 includes cement pockets 44 in anchoring side 48, which correspond to depressions 58 provided in upper side 46 of intermediate component 24 (Fig. 3). The size and shape of cement pockets 44 differ from the size and shape of depressions 58 as a result of the bending operation. This size difference may be considered and accounted for in the size and shape of protrusions 26 of the male die plate 20 of the forging die. When the femoral component 50 is anchored to the resected femur, cement pockets 44 are filled with bone cement. The cement may also be applied to regions of anchoring side 48 which have no cement pockets.

Finally, pre-finished component 38 is subjected to finishing steps, such as polishing and cleaning, to form the final femoral component. Features of pre-finished component 38 are similar to the features of the final femoral component except for changes resulting from these finishing steps.

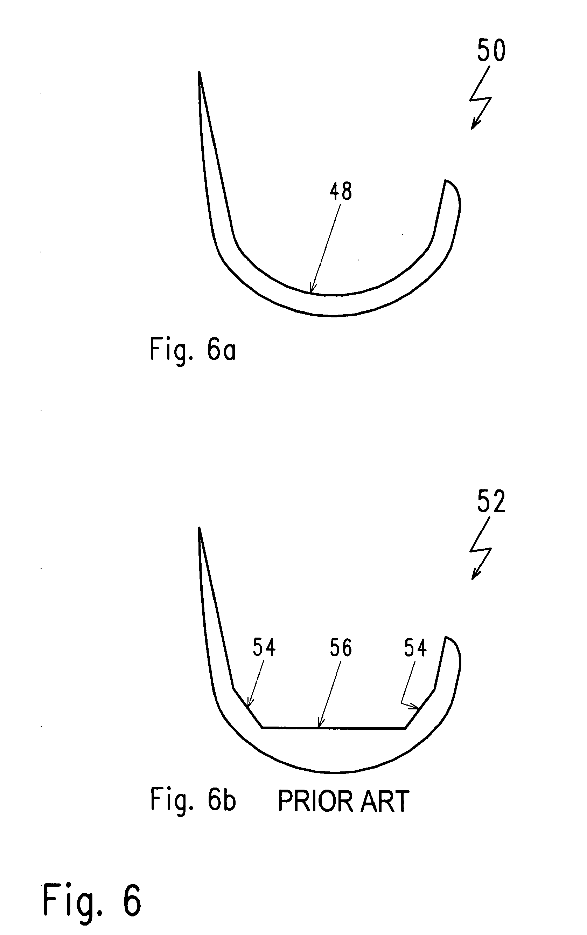

As can be seen in Fig. 6a, the bending operation or step includes forming anchoring side 48 of femoral component 50 such that it is substantially rounded and free of chamfers, because punch 40 is provided with a correspondingly rounded profile. Anchoring side 48 of femoral component 50 is adapted, such as through its shape and size, to directly contact the resected femur.

As can be seen in Fig. 6b, known femoral components 52 have an anchoring side 56 with two or more chamfers or angular seams 54 along the anterior-posterior direction. As a consequence, the thickness of non-chamfered femoral component 50 in the distal region of femoral component 50 may be smaller than the corresponding thickness of known femoral component 52. In addition, the thickness of femoral component 50 is substantially constant along the anterior-posterior direction of femoral component 50.

Advantageously, formation of the cement pockets as part of the forging operation saves an additional drilling or milling step. For example, if cement pockets 44 are not formed in the forging operation as described above, a drilling or milling step would be necessary to form cement pockets. This step is complicated by restricted access to the anterior or posterior portions of the anchoring side of the femoral component, because the other portion is arranged oppositely to the accessed portion.

While the present invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. The invention in its broader aspects is therefore not limited to the specific details, representative apparatus and method and illustrative examples shown and described. Accordingly, departures may be made from such details without departing from the scope of the general inventive concept.

List of Reference Numerals:
- 10: right femur
- 12: lateral condyle
- 14: medial condyle
- 16: facies patellaris
- 18: blank
- 20: male die plate
- 22: female die plate
- 24: intermediate component
- 26: protrusion
- 28: cavity
- 30: anterior portion
- 32: distal portion
- 34: lateral condyle portion
- 36: medial condyle portion
- 38: pre-finished component
- 40: punch
- 42: die plate
- 44: cement pocket
- 46: upper side
- 48: anchoring side
- 50: femoral component
- 52: femoral component
- 54: chamfer
- 56: anchoring side
- 58: depression

## Claims

1. Amethod for manufacturing an orthopedic implant, the method comprising:
providing a blank (18) adapted to be formed into the orthopedic implant;
forging said blank (18) to form an intermediate component (24); and
bending said intermediate component (24) to form a pre-finished component (38).

2. The method of claim 1, wherein said intermediate component (24) has a substantially planar shape.

3. The method of claim 1 or 2, wherein said pre-finished component (38) has a shape substantially corresponding to said shape of the orthopedic implant.

4. The method of any of the preceding claims, wherein said step of forging said blank (18) includes forming at least one depression (58) in a first side (46) of said blank (18),
in particular wherein said depression (58) forms a cement pocket (44) in the orthopedic implant, and/or said first side serves (46) as an anchoring side (48) of the orthopedic implant arrangeable at a resection surface of a bone.

5. The method of claim 4, wherein a shape and size of said depression (58) in said intermediate component (18) is different from a shape and size of said cement pocket (44) in the orthopedic implant.

6. The method of claim 4 or 5, wherein said step of forming said depression (58) further includes accounting for an effect of the step of bending said intermediate component (24) on the shape and size of said depression (58).

7. The method of any of the preceding claims, wherein said step of forging said blank (18) comprises a die forging operation,
and/or wherein said step of bending said intermediate component (24) comprises a die bending operation.

8. The method of any of the preceding claims, wherein said step of bending said intermediate component (24) includes forming a substantially rounded anchoring side (48) of said orthopedic implant, in particular such that said orthopaedic implant has a substantially uniform thickness.

9. The method of any of the preceding claims, wherein said blank (18) comprises a pre-forged blank.

10. The method of any of the preceding claims, wherein said blank (18) is formed from at least one of titanium and titanium alloy.

11. The method of any of the preceding claims, wherein the orthopedic implant is a femoral component (50) of a knee joint prosthesis.

12. An orthopedic implant, in particular a femoral component (50) of a knee joint prosthesis, obtained or obtainable by a method according to any of the preceding claims.

13. The orthopedic implant of claim 12, wherein said orthopedic implant has a substantially uniform thickness.

14. The orthopedic implant of claim 12 or 13, wherein said orthopedic implant comprises a substantially rounded anchoring side (48), which in particular is free of chamfers and/or angular seams.

15. The orthopedic implant of any of claims 12 to 14, wherein a first side (46), in particular an anchoring side (48), of said orthopedic implant is provided with at least one depression (44), in particular a cement pocket.
